# EUROPEAN PATENT SPECIFICATION

(11) **EP 1 480 609 B1**
(45) Date of publication and mention of the grant of the patent: **23.07.2014**
(21) Application number: 03710261.3
(22) Date of filing: 06.03.2003
(51) Int. Cl.: A61K 8/67, A61Q 19/02

(54) **WHITENING COSMETIC COMPOSITION**
KOSMETISCHES DEPIGMENTIERUNGSMITTEL
COMPOSITION COSMETIQUE BLANCHISSANTE

(30) Priority: 06.03.2002 JP 2002061120; 12.03.2002 US 363102 P
(43) Date of publication of application: 01.12.2004
(73) Proprietor: Showa Denko K.K., Tokyo 105-8518 (JP)
(72) Inventor: KATO, Eiko, Chiba-shi Chiba 267-0056 (JP); OGATA, Eiji, Chiba-shi Chiba 267-0056 (JP); YONEDA, Tadashi, Chiba-shi Chiba 267-0056 (JP); TAKATA, Jiro, Fukuoka-shi, Fukuoka 819-0044 (JP)
(74) Representative: Strehl Schübel-Hopf & Partner
(86) International application number: PCT/JP2003/002646
(87) International publication number: WO 2003/074019

(56) References cited:
- WO-A-98/51679
- WO-A-03/037290
- US-A- 2 988 553
- US-A- 5 932 612
- PATENT ABSTRACTS OF JAPAN vol. 014, no. 397 (C-0752), 28 August 1990 (1990-08-28) & JP 02 149577 A (EISAI CO LTD), 8 June 1990 (1990-06-08)
- JIRO TAKATA ET AL: "PRODRUGS OF VITAMIN E.1" JOURNAL OF PHARMACEUTICAL SCIENCES, AMERICAN PHARMACEUTICAL ASSOCIATION. WASHINGTON, US, vol. 84, no. 1, 1995, pages 96-100, XP002092803 ISSN: 0022-3549
- DATABASE CA [Online] CHEMICAL ABSTRACTS SERVICE, COLUMBUS, OHIO, US; AOKI, YUZURU ET AL: "Skin cosmetics containing vitamin E derivatives and Citrus junos extracts" retrieved from STN Database accession no. 134:256613 CA XP002250037 & JP 2001 081022 A (KANEBO, LTD., JAPAN) 27 March 2001 (2001-03-27)

## Description

### TECHNICAL FIELD

The present invention relates to the external use of a tocopherol alkylglycine ester and/or a salt thereof, as an active ingredient, exhibiting effects of preventing pigmentation or eliminating formed pigments in the skin, for cosmetic skin whitening.

### BACKGROUND ART

Heretofore, many preparations for external use on skin have been proposed and have been employed, in order to reduce pigmentation of the skin, such as spots and freckles due to exposure to UV rays, or darkening observed after wounds or burns heal or observed after healing surgical operation wounds.

For example, polyphenols are widely known as reducing decoloration effects of melanin pigments. In particular, hydroquinone is widely employed in the field of clinical medicine in USA. However, hydroquinone is pointed out as being strongly irritating on the skin, and for this reason, it is difficult to employ it as a preparation for external use on skin, having a satisfactory level of safety.

In addition, vitamin C or derivatives thereof such as ascorbyl 2-phosphate or ascorbyl 2-glucoside have been widely employed as pigmentation preventive agents in cosmetic compositions. However, according to our knowledge, the effects of preventing pigmentation of the ascorbic acids described above are weak, and they cannot be believed to be effective in view of many aspects. Furthermore, they exhibit superior effects of preventing pigmentation, but the effects of reducing deposition of the formed pigments may or may not be sufficient.

Tocopherols, also known as vitamin E (such as α-tocopherol, β-tocopherol, γ-tocopherol, and δ-tocopherol), and derivatives thereof such as tocopherol acetate and tocopherol nicotinate, are known to exhibit effects such as antioxidation effects, effects of stabilizing vital membranes, immune activating effects, and effects of accelerating blood circulation, and have heretofore been added to medicines, cosmetics, and the like; however, there has been no reference to their effects of preventing pigmentation or eliminating formed pigments. As for tocopherols, there is an example of their effect as a remedy for pigmentation by their oral administration (K. Werininghaus et al., Arch Dermatol, 130, 1257, 1997). In addition, there are examples of effects of a specific derivative, tocopherol ferulic acid ester, in inhibiting pigmentation of cultured cells (M. Ichihashi et al., Anticancer Res., 19, 3769, 1999, and the like). However, effects of preventing pigmentation or eliminating formed pigments by dermal application of tocopherols have not been reported.

In addition, in order to avoid difficulty in view of formulation of the oil-soluble tocopherol derivatives described above, water-soluble tocopherol phosphates, amino acid esters, or alkylaminocarbonates have been proposed.

Database CA [Online] CHEMICAL ABSTRACTS SERVICE, COLUMBUS, OHIO, US; AOKI YUZURU ET AL: "Skin cosmetics containing vitamin E derivatives and Citrus junos extracts" XP002250037 retrieved from STN Database accession no. 134:256613 CA, US-A-5 932 612, and WO 98/51679 A disclose tocopherol and derivatives thereof including esters for lightening or depigmentating the skin.

### DISCLOSURE OF INVENTION

Considering these circumstances, an object of the present invention is to provide a preparation for external use on skin having a high level of safety and comprising an active ingredient exhibiting superior effects of preventing skin pigmentation or eliminating formed pigments in the skin, and in particular, to provide a cosmetic composition exhibiting superior so-called "whitening effects".

As a result of diligent research in order to achieve this object, the present inventors discovered that tocopherol alkylglycine esters and salts thereof exhibit strong effects of preventing skin pigmentation and eliminating formed pigments in the skin, thus completing the present invention.

That is, the present invention relates to the features described below.
[1] The external use of a tocopherol alkylglycine ester and/or a salt thereof for cosmetic skin whitening,
   wherein the tocopherol alkylglycine ester is a compound represented by formula (1) described below: 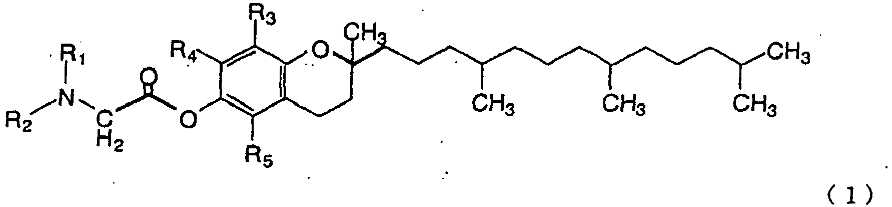 wherein R₁ and R₂ represent the same or different lower alkyl group or a hydrogen atom; and R₃, R₄, and R₅ represent a hydrogen atom or a methyl group; with the proviso that R₁ and R₂ do not represent a hydrogen atom at the same time.
[2] The use according to [1] described above, wherein the tocopherol alkylglycine ester is at least one compound selected from an α-tocopherol alkylglycine ester, a γ-tocopherol alkylglycine ester, and a δ-tocopherol alkylglycine ester.
[3] The use according to [1] described above, wherein the tocopherol alkylglycine ester is an α-tocopherol alkylglycine ester.
[4] The use according to [3] described above, characterized in that the α-tocopherol alkylglycine ester is a dl-α-tocopherol alkylglycine ester.
[5] The use according to [3] described above, characterized in that the α-tocopherol alkylglycine ester is a d-α-tocopherol alkylglycine ester.
[6] The use according to [1], described above, characterized in that the tocopherol alkylglycine ester is a γ-tocopherol alkylglycine ester.
[7] The use according to [6] described above, characterized in that the γ-tocopherol alkylglycine ester is a d-γ-tocopherol alkylglycine ester.
[8] The use according to [1], described above, characterized in that the tocopherol alkylglycine ester is a δ-tocopherol alkylglycine ester.
[9] The use according to [8] described above, characterized in that the δ-tocopherol alkylglycine ester is a d-δ-tocopherol alkylglycine ester.
[10] The use according to [1] described above, characterized in that the tocopherol alkylglycine ester is tocopherol dimethylglycine ester.
[11] The use according to [1] described above; characterized in that the salt of a tocopherol alkylglycine ester is a salt of an organic acid or an inorganic acid.
[12] The use according to [11] described above, wherein the salt of a tocopherol alkylglycine ester is hydrochloride.
[13] The use according to [1] described above, wherein an added amount of the tocopherol alkylglycine ester and/or the salt thereof ranges from 0.1 to 10% by mass.

### BEST MODE FOR CARRYING OUT THE INVENTION

First, a tocopherol alkylglycine ester and a salt thereof which are the ingredients employed in the present invention are described.

A tocopherol alkylglycine ester employed in the present invention is employed as an active ingredient having effects of preventing pigmentation or eliminating formed pigments in the skin, and is a compound represented by, for example, a formula (1) described below: 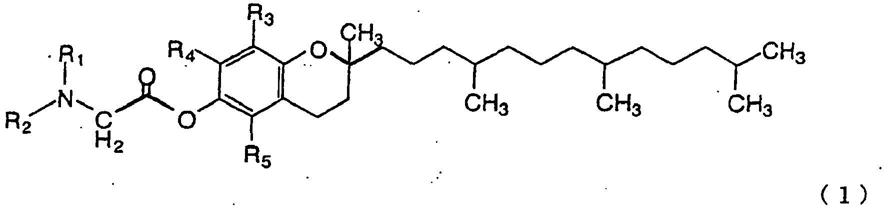 wherein R₁ and R₂ represent the same or different lower alkyl group or a hydrogen atom; and R₃ , R₄, and R₅ represent a hydrogen atom or a methyl group; with the proviso that R₁ and R₂ do not represent a hydrogen atom at the same time.

The term "lower alkyl group" described in the definition of R₁ and R₂ means a straight-chain or branched alkyl group having 1 to 6 carbon atoms. As examples thereof, mention may be made of methyl, ethyl, n-propyl, n-butyl, isopropyl, isobutyl, 1-methylpropyl, tert-butyl, n-pentyl, 1-ethylpropyl, isoamyl, n-hexyl, and the like. The most preferable group among these is a methyl group.

As a salt of the compound used in the present invention, salts are not particularly restricted, so long as they are salts of an organic acid or an inorganic acid. As preferable examples thereof, mention may be made of a hydrohalogenic acid salt, an organic acid salt, and the like. Among these, hydrochloride is preferable since it is advantageous in that the solubility in water increases and handling is facilitated due to its powdered form.

The tocopherol derivatives represented by the formula (1) described above have an asymmetric carbon at the 2-position of the chroman ring. For this reason, there are stereomers such as d-form, 1-form, and dl-form. It should be understood that the present invention includes all the isomers described above.

The compounds used in the present invention may be produced by conventional production methods, and an example thereof is described in the following.

The compounds can be easily produced by performing an esterification reaction between a tocopherol represented by the formula (2) described below: 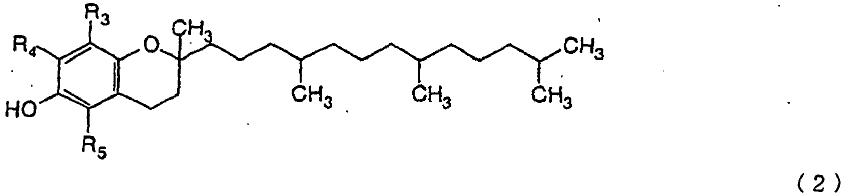 wherein R₃, R₄, and R₅ represent a hydrogen atom or a methyl group,
and any one of an alkylglycine represented by the formula (3) described below: 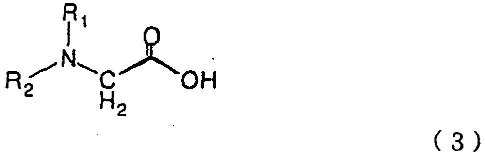 wherein R₁ and R₂ represent an identical or different lower alkyl group or a hydrogen atom; with the proviso that R₁ and R₂ do not represent a hydrogen atom at the same time, a reactive acid derivative thereof, and a hydrohalogenic acid salt thereof, in a conventional manner.

In the case of directly performing the esterification using a free alkylglycine, usually, the reaction is preferably performed in the presence of an active esterification reagent such as dicyclohexylcarbodiimide or N,N-disuccinimide oxalate. The solvent in the reaction is most preferably pyridine.

In addition, in a reaction with a reactive acid derivative, a reaction employing an acyl halide, and in particular, an acyl chloride is preferable.

In the case of producing a salt of a tocopherol alkylglycine ester, the hydrohalogenic acid salt may be produced by once producing an ester form, and subsequently adding an acid thereto according to a conventional method, thus forming a salt, or alternatively, may be produced by previously employing a salt of an alkylglycine as a starting material.

The present invention relates to the external use of a tocopherol alkylglycine ester and a salt thereof for cosmetic skin whitening.

The whitening cosmetic composition can be produced by adding 0.1 to 10% by mass and preferably 0.5 to 2% by mass of a tocopherol alkylglycine ester and/or a salt thereof to an alcohol such as ethanol or propylene glycol; preservatives such as methyl parahydroxybenzoate, ethyl parahydroxybenzoate, butyl parahydroxybenzoate, or propyl parahydroxybenzoate; purified water; and the like.

Examples of whitening cosmetic compositions include, for example, skin milk, skin cream, foundation cream, cream for massaging, cleansing cream, lotion, pack, ointment, bath preparation, body soap, and the like. The kinds thereof are not restricted, so long as they come into contact with skin during use. They may be in the form of gel. In addition, the user may be any user irrespective of sex or age.

In addition, in the whitening cosmetic compositions, ingredients other than those described above, which are commonly employed in whitening cosmetic compositions, can be added within a range which does not impair the effects of the present invention.

### EXAMPLES

In the following, the present invention is described in detail by referring to Examples. In the Examples, the added amount is based on % by mass.

### Example of Synthesis

To a solution of 59.1 g of dicyclohexylcarbodiimide and 40.0 g of N,N-dimethylglycine hydrochloride dissolved in 320 g of pyridine, a solution of 60.0 g of dl-α-tocopherol dissolved in 240 g of pyridine was added. The mixture was allowed to react for 8 hours while stirring at room temperature, and pyridine was distilled out. 2000 ml of water and 1000 ml of ethyl acetate were added to the residue, and about 40 g of sodium carbonate was added thereto to adjust the pH to between 7 and 8. The ethyl acetate phase was separated. Extraction from the water phase was carried out three time using 200 ml of ethyl acetate each time. The ethyl acetate phase which was separated and the ethyl acetate phases used for extraction were combined, and the ethyl acetate was distilled off. Ethyl acetate was added to the residue, the mixture was dried over anhydrous sodium sulfate, and solid substances were filtered out. The concentration of dl-α-tocopherol dimethylglycine ester in the filtrate was adjusted to between 7 to 8%. 20% hydrochloric acid in dioxane was added thereto for neutralization in an amount such that the molar amount of the hydrochloric acid is 1.5 times of that of the dl-α-tocopherol dimethylglycine ester. Solid substance obtained was collected by filtration, and recrystallized in methanol/acetone solvent to yield 49.2 g of dl-α-tocopherol dimethylglycine ester hydrochloride.

### Example 1

### Lotion 1

Ingredients 1) to 4) were uniformly dispersed and dissolved so as to obtain the final concentrations described below, and subsequently the resulting mixture was added to ingredient 5) with stirring, thus producing Lotion 1.

| | | |
|---|---|---|
| 1) | dl-α-tocopherol dimethylglycine ester hydrochloride | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### Lotion 2

Ingredients 1) to 4) were uniformly dispersed and dissolved so as to obtain the final concentrations described below, and subsequently the resulting mixture was added to ingredient 5) with stirring, thus producing Lotion 2.

| | | |
|---|---|---|
| 1) | d-α-tocopherol dimethylglycine ester hydrochloride | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### Lotion 3

Ingredients 1) to 4) were uniformly dispersed and dissolved so as to obtain the final concentrations described below, and subsequently the resulting mixture was added to ingredient 5) with stirring, thus producing Lotion 3.

| | | |
|---|---|---|
| 1) | d-γ-tocopherol dimethylglycine ester hydrochloride | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### Lotion 4

Ingredients 1) to 4) were uniformly dispersed and dissolved so as to obtain the final concentrations described below, and subsequently the resulting mixture was added to ingredient 5) with stirring, thus producing Lotion 4.

| | | |
|---|---|---|
| 1) | d-δ-tocopherol dimethylglycine ester hydrochloride | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### Lotion 5 (Comparative control)

Ingredients 1) to 4) were uniformly dispersed and dissolved so as to obtain the final concentrations described below, and subsequently the resulting mixture was added to ingredient 5) with stirring, thus producing Lotion 5.

| | | |
|---|---|---|
| 1) | Sodium ascorbyl 2-phosphate | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

### Lotion 6 (Negative control)

Ingredients 1) to 4) were uniformly dispersed and dissolved so as to obtain the final concentrations described below, and subsequently the resulting mixture was added to ingredient 5) with stirring, thus producing Lotion 6.

| | | |
|---|---|---|
| 1) | Purified water | 2.00 |
| 2) | Ethanol | 5.00 |
| 3) | Propylene glycol | 5.00 |
| 4) | Methyl parahydroxybenzoate | 0.20 |
| 5) | Purified water | 87.8 |

All lotions described above were uniformly dissolved and exhibited good stability over time.

### Example 2

### Lotion 7

Ingredients 1) to 4) were uniformly dispersed and dissolved so as to obtain the final concentrations described below, and subsequently the resulting mixture was added to ingredient 5) with stirring, thus producing Lotion 7.

| | | |
|---|---|---|
| 1) | dl-α-tocopherol dimethylglycine ester hydrochloride | 0.10 |
| 2) | Ethanol | 5.00 |
| 3) | Propylene glycol | 5.00 |
| 4) | Methyl parahydroxybenzoate | 0.20 |
| 5) | Purified water | 89.7 |

### Lotion 8

Ingredients 1) to 4) were uniformly dispersed and dissolved so as to obtain the final concentrations described below, and subsequently the resulting mixture was added to ingredient 5) with stirring, thus producing Lotion 8.

| | | |
|---|---|---|
| 1) | d-α-tocopherol dimethylglycine ester hydrochloride | 0.10 |
| 2) | Ethanol | 5.00 |
| 3) | Propylene glycol | 5.00 |
| 4) | Methyl parahydroxybenzoate | 0.20 |
| 5) | Purified water | 89.7 |

### Lotion 9

Ingredients 1) to 4) were uniformly dispersed and dissolved so as to obtain the final concentrations described below, and subsequently the resulting mixture was added to ingredient 5) with stirring, thus producing Lotion 9.

| | | |
|---|---|---|
| 1) | d-γ-tocopherol dimethylglycine ester hydrochloride | 0.10 |
| 2) | Ethanol | 5.00 |
| 3) | Propylene glycol | 5.00 |
| 4) | Methyl parahydroxybenzoate | 0.20 |
| 5) | Purified water | 89.7 |

### Lotion 10

Ingredients 1) to 4) were uniformly dispersed and dissolved so as to obtain the final concentrations described below, and subsequently the resulting mixture was added to ingredient 5) with stirring, thus producing Lotion 10.

| | | |
|---|---|---|
| 1) | d-δ-tocopherol dimethylglycine ester hydrochloride | 0.10 |
| 2) | Ethanol | 5.00 |
| 3) | Propylene glycol | 5.00 |
| 4) | Methyl parahydroxybenzoate | 0.20 |
| 5) | Purified water | 89.7 |

### Lotion 11 (Comparative control)

Ingredients 1) to 4) were uniformly dispersed and dissolved so as to obtain the final concentrations described below, and subsequently the resulting mixture was added to ingredient 5) with stirring, thus producing Lotion 11.

| | | |
|---|---|---|
| 1) | Sodium ascorbyl 2-phosphate | 0.10 |
| 2) | Ethanol | 5.00 |
| 3) | Propylene glycol | 5.00 |
| 4) | Methyl parahydroxybenzoate | 0.20 |
| 5) | Purified water | 89.7 |

### Lotion 12 (Negative control)

Ingredients 1) to 4) were uniformly dispersed and dissolved so as to obtain the final concentrations described below, and subsequently the resulting mixture was added to ingredient 5) with stirring, thus producing Lotion 12.

| | | |
|---|---|---|
| 1) | Purified water | 0.10 |
| 2) | Ethanol | 5.00 |
| 3) | Propylene glycol | 5.00 |
| 4) | Methyl parahydroxybenzoate | 0.20 |
| 5) | Purified water | 89.7 |

All lotions described above were uniformly dissolved and exhibited good stability over time.

### Example 3

### Gel composition 1

Ingredient 1) was uniformly dispersed in ingredient 2) so as to obtain the final concentrations described below, and subsequently the resulting mixture was added to ingredient 3) with stirring, thus producing the desired gel composition 1.

| | | |
|---|---|---|
| 1) | dl-α-tocopherol dimethylglycine ester hydrochloride | 10 |
| 2) | Glycerol | 20 |
| 3) | Octyldodecyl myristate | 70 |

### Gel composition 2

Ingredient 1) was uniformly dispersed in ingredient 2) so as to obtain the final concentrations described below, and subsequently the resulting mixture was added to ingredient 3) with stirring, thus producing the desired gel composition 2.

| | | |
|---|---|---|
| 1) | d-α-tocopherol dimethylglycine ester hydrochloride | 10 |
| 2) | Glycerol | 20 |
| 3) | Octyldodecyl myristate | 70 |

### Gel composition 3

Ingredient 1) was uniformly dispersed in ingredient 2) so as to obtain the final concentrations described below, and subsequently the resulting mixture was added to ingredient 3) with stirring, thus producing the desired gel composition 3.

| | | |
|---|---|---|
| 1) | d-γ-tocopherol dimethylglycine ester hydrochloride | 10 |
| 2) | Glycerol | 20 |
| 3) | Octyldodecyl myristate | 70 |

### Gel composition 4

Ingredient 1) was uniformly dispersed in ingredient 2) so as to obtain the final concentrations described below, and subsequently the resulting mixture was added to ingredient 3) with stirring, thus producing the desired gel composition 4.

| | | |
|---|---|---|
| 1) | d-δ-tocopherol dimethylglycine ester hydrochloride | 10 |
| 2) | Glycerol | 20 |
| 3) | Octyldodecyl myristate | 70 |

### Gel composition 5 (Comparative control)

Ingredient 1) was uniformly dispersed in ingredient 2) so as to obtain the final concentrations described below, and subsequently the resulting mixture was added to ingredient 3) with stirring, thus producing the desired gel composition 5.

| | | |
|---|---|---|
| 1) | Sodium ascorbyl 2-phosphate | 10 |
| 2) | Glycerol | 20 |
| 3) | Octyldodecyl myristate | 70 |

### Gel composition 6 (Negative control)

Ingredient 1) was uniformly dispersed in ingredient 2) so as to obtain the final concentrations described below, and subsequently the resulting mixture was added to ingredient 3) with stirring, thus producing the desired gel composition 6.

| | | |
|---|---|---|
| 1) | Purified water | 10 |
| 2) | Glycerol | 20 |
| 3). | Octyldodecyl myristate | 70 |

All gel compositions described above were uniformly dissolved, and exhibited good stability over time.

### Example 4

### Effects of preventing pigmentation

Hair on the entire surface of the back of each of 50 male Wiser Maple guinea pigs (WM, SPF), 7 weeks old, was cut by means of electric hair clippers (0.05 mm blade), and was subsequently shaved by means of an electric shaver, followed by covering with an adhesive stretch bandage (SILKYTEX, covered on the outside thereof with an aluminum foil) wherein 6 holes of 1.5 cm x 1.5 cm had been formed.

Each product of the Lotions 1 to 12 and the Gel compositions 1 to 6, prepared in Examples 1 to 3, in an amount of 0.05 ml, was successively applied to 10 holes described above.

Four hours after the application, the application part was cleaned with absorbent cotton which contained water, followed by drying. Subsequently, the animal was held by a retainer, followed by exposure of UV rays having medium wavelength (UVB) with 300 mJ/cm² to each part from a distance of approximately 10 cm by means of a UV exposure apparatus (Shinano Co., Ltd., Toshiba FL40S/E30 model fluorescent lamp, equipped with six SE lamps).

After the exposure, 0.05 ml of each of the same Lotions 1 to 12 and Gel compositions 1 to 6 as described above was again applied to the corresponding part.

The operation described above was repeated for 3 days. Fourteen days after the final exposure, the strength of pigmentation was evaluated by evaluation points according to the evaluation criteria described in the following. Furthermore, the lightness of the skin at 5 spots in total, that is, the four corners and the center part of each of the applied and exposed parts was measured by means of a color-difference meter (Minolta Co., Ltd., CR-20).

The effects of preventing pigmentation of each of the products were determined by the average value of the evaluation points (10 pieces of data per product) and the average value of the lightness (50 pieces of data per product).

**Evaluation criteria of pigmentation**

| | |
|---|---|
| No pigmentation is observed | evaluation point 0 |
| Very slight pigmentation is observed | evaluation point 1 |
| Slight pigmentation is observed | evaluation point 2 |
| Fair degree of pigmentation is observed | evaluation point 3 |
| Strong pigmentation is observed | evaluation point 4 |

**Results (average value)**

| Product | Evaluation point | Lightness |
|---|---|---|
| Lotion 1 | 0.8 | 63.0 |
| Lotion 2 | 0.7 | 63.1 |
| Lotion 3 | 0.6 | 62.8 |
| Lotion 4 | 0.5 | 64.0 |
| Lotion 5 | 2.1 | 61.8 |
| Lotion 6 | 3.0 | 59.5 |
| Lotion 7 | 2.0 | 61.7 |
| Lotion 8 | 2.0 | 60.7 |
| Lotion 9 | 1.5 | 62.0 |
| Lotion 10 | 1.7 | 62.0 |
| Lotion 11 | 3.0 | 59.9 |
| Lotion 12 | 3.0 | 60.1 |
| Gel composition 1 | 0.4 | 63.8 |
| Gel composition 2 | 0.4 | 62.9 |
| Gel composition 3 | 0.4 | 63.1 |
| Gel composition 4 | 0.4 | 63.0 |
| Gel composition 5 | 1.2 | 62.1 |
| Gel composition 6 | 3.0 | 59.9 |

As is apparent from the results described above, in each of the Lotions 1 to 4 and 7 to 10, and the Gel compositions 1 to 4, according to the present invention, superior effects of preventing pigmentation were observed.

### Example 5

### Effects of eliminating pigmentation

Hair on the entire surface of the back of each of 50 male Wiser Maple guinea pigs (WM, SPF), 6 weeks old, was cut by means of electric hair clippers (0.05 mm blade), and was subsequently shaved by means of an electric shaver, followed by covering with an adhesive stretch bandage (SILKYTEX, covered the outside thereof with an aluminum foil) wherein 6 holes of 1.5 cm x 1.5 cm had been formed. Subsequently, the animal was held by a retainer, followed by exposure of UV rays having medium wavelength (UVB) of 750 mJ/cm² to each part from a distance of approximately 10 cm by means of a UV exposure apparatus (Shinano Co., Ltd., Toshiba FL40S/E30 model fluorescent lamp, equipped with six SE lamps).

From 4 days after the exposure to 28 days, each product of the Lotions 1 to 12 and the Gel compositions 1 to 6, prepared in Examples 1 to 3, in an amount of 0.05 ml, was successively applied to 10 holes described above, twice a day, that is, in the morning and in the evening.

Twenty-eight days after the exposure, the strength of pigmentation was evaluated by evaluation points according to the same evaluation criteria as described in the Example. The effects of preventing pigmentation were evaluated by the average value obtained from the evaluation points (10 data per product).

**Results (average value)**

| Product | Evaluation point |
|---|---|
| Lotion 1 | 2.2 |
| Lotion 2 | 2.2 |
| Lotion 3 | 2.0 |
| Lotion 4 | 2.0 |
| Lotion 5 | 3.3 |
| Lotion 6 | 3.5 |
| Lotion 7 | 3.0 |
| Lotion 8 | 3.0 |
| Lotion 9 | 3.0 |
| Lotion 10 | 3.0 |
| Lotion 11 | 3.5 |
| Lotion 12 | 3.8 |
| Gel composition 1 | 2.2 |
| Gel composition 2 | 2.1 |
| Gel composition 3 | 2.0 |
| Gel composition 4 | 1.9 |
| Gel composition 5 | 2.9 |
| Gel composition 6 | 3.5 |

As is apparent from the results described above, even in each of the Lotions (1 to 4 and 7 to 10) and Gel compositions (1 to 4) according to the present invention, superior effects of eliminating pigmentation were observed.

### INDUSTRIAL APPLICABILITY

The compositions used in the present invention have a high level of safety, and exhibit superior effects of preventing pigmentation or eliminating formed pigments in the skin. For these reasons, they are useful as cosmetic compositions exhibiting superior so-called whitening effects.

## Claims

1. The external use of a tocopherol alkylglycine ester and/or a salt thereof for cosmetic skin whitening,
wherein the tocopherol alkylglycine ester is a compound represented by a formula (1) described below: 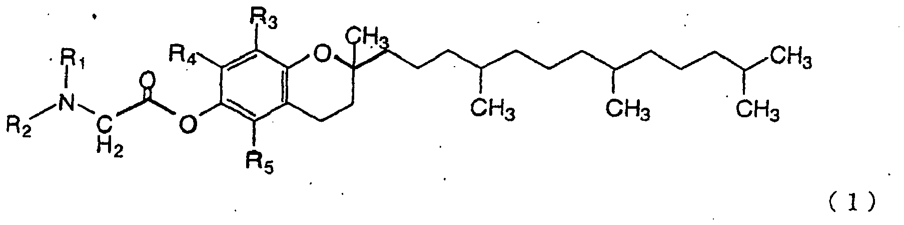 wherein R₁ and R₂ represent the same or different lower alkyl group or a hydrogen atom; and R₃, R₄, and R₅ represent a hydrogen atom or a methyl group; with the proviso that R₁ and R₂ do not represent a hydrogen atom at the same time.

2. The use according to Claim 1, wherein the tocopherol alkylglycine ester is at least one compound selected from an α-tocopherol alkylglycine ester, a γ-tocopherol alkylglycine ester, and a δ-tocopherol alkylglycine ester.

3. The use according to Claim 1, wherein the tocopherol alkylglycine ester is an α-tocopherol alkylglycine ester.

4. The use according to Claim 3, **characterized in that** the α-tocopherol alkylglycine ester is a dl-α-tocopherol alkylglycine ester.

5. The use according to Claim 3, **characterized in that** the α-tocopherol alkylglycine ester is a d-α-tocopherol alkylglycine ester.

6. The use according to Claim 1, **characterized in that** the tocopherol alkylglycine ester is a γ-tocopherol alkylglycine ester.

7. The use according to Claim 6, **characterized in that** the γ-tocopherol alkylglycine ester is a d-γ-tocopherol alkylglycine ester.

8. The use according to Claim 1, **characterized in that** the tocopherol alkylglycine ester is a δ-tocopherol alkylglycine ester.

9. The use according to Claim 8, **characterized in that** the δ-tocopherol alkylglycine ester is a d-δ-tocopherol alkylglycine ester.

10. The use according to Claim 1, **characterized in that** the tocopherol alkylglycine ester is 5 tocopherol dimethylglycine ester.

11. The use according to Claim 1, **characterized in that** the salt of a tocopherol alkylglycine ester is a salt of an organic acid or an inorganic acid.

12. The use according to Claim 11, wherein the salt of a tocopherol alkylglycine ester is hydrochloride.

13. The use according to Claim 1, wherein an added amount of the tocopherol alkylglycine ester and/or the salt thereof ranges from 0.1 to 10% by mass.

## Patentansprüche

1. Äußere Verwendung eines Tocopherolalkylglycinesters und/oder eines Salzes davon für kosmetisches Hautbleichen, wobei der Tocopherolalkylglycinester eine Verbindung der nachstehenden Formel (1) ist: 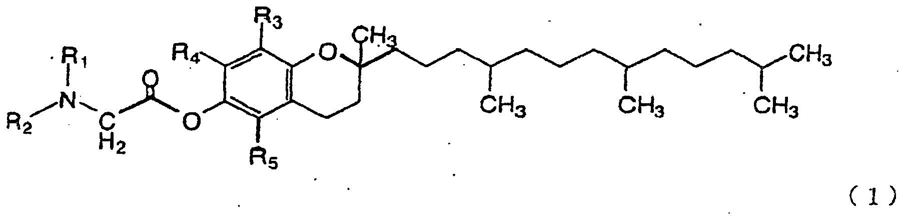 worin R₁ und R₂ dieselbe oder unterschiedliche Niederalkylgruppe oder ein Wasserstoffatom darstellen; und R₃, R₄ und R₅ ein Wasserstoffatom oder eine Methylgruppe darstellen; mit der Maßgabe, dass R₁ und R₂ nicht gleichzeitig ein Wasserstoffatom darstellen.

2. Verwendung nach Anspruch 1, wobei der Tocopherolalkylglycinester mindestens eine Verbindung ist, die unter einem α-Tocopherolalkylglycinester, einem γ-Tocopherolalkylglycinester und einem δ-Tocopherolalkylglycin-ester ausgewählt ist.

3. Verwendung nach Anspruch 1, wobei der Tocopherolalkylglycinester ein α-Tocopherolalkylglycinester ist.

4. Verwendung nach Anspruch 3, **dadurch gekennzeichnet, dass** der α-Tocopherolalkylglycinester ein dl-α-Tocopherolalkylglycinester ist.

5. Verwendung nach Anspruch 3, **dadurch gekennzeichnet, dass** der α-Tocopherolalkylglycinester ein d-α-Tocopherolalkylglycinester ist.

6. Verwendung nach Anspruch 1, **dadurch gekennzeichnet, dass** der Tocopherolalkylglycinester ein γ-Tocopherolalkylglycinester ist.

7. Verwendung nach Anspruch 6, **dadurch gekennzeichnet, dass** der γ-Tocopherolalkylglycinester ein d-γ-Tocopherolalkylglycinester ist.

8. Verwendung nach Anspruch 1, **dadurch gekennzeichnet, dass** der Tocopherolalkylglycinester ein δ-Tocopherolalkylglycinester ist.

9. Verwendung nach Anspruch 8, **dadurch gekennzeichnet, dass** der δ-Tocopherolalkylglycinester ein d-δ-Tocopherolalkylglycinester ist.

10. Verwendung nach Anspruch 1, **dadurch gekennzeichnet, dass** der Tocopherolalkylglycinester Tocopheroldimethylglycinester ist.

11. Verwendung nach Anspruch 1, **dadurch gekennzeichnet, dass** das Salz eines Tocopherolalkylglycinesters ein Salz einer organischen Säure oder einer anorganischen Säure ist.

12. Verwendung nach Anspruch 11, wobei das Salz eines Tocopherolalkylglycinesters ein Hydrochlorid ist.

13. Verwendung nach Anspruch 1, wobei die zugegebene Menge des Tocopherolalkylglycinesters und/oder des Salzes davon im Bereich von 0,1 bis 10 Mass.-% ist.

## Revendications

1. Utilisation externe d'un ester de tocophérol alkylglycine et/ou d'un sel de celui-ci pour un blanchiment de peau cosmétique, dans lequel l'ester de tocophérol alkylglycine est un composé représenté par une formule (1) décrite ci-dessous : 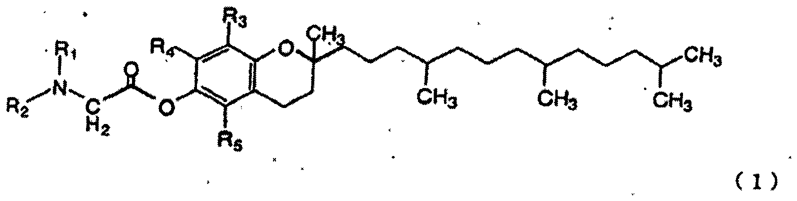 dans laquelle R¹ et R² représentent un groupe alkyle inférieur identique ou différent ou un atome d'hydrogène ; et R₃, R₄ et R₅ représentent un atome d'hydrogène ou un groupe méthyle ; à condition que R₁ et R₂ ne représentent pas un atome d'hydrogène simultanément.

2. Utilisation selon la revendication 1, dans lequel l'ester de tocophérol alkylglycine est au moins un composé choisi parmi un ester d'α-tocophérol alkylglycine, un ester de γ-tocophérol alkylglycine, et un ester de δ-tocophérol alkylglycine.

3. Utilisation selon la revendication 1, dans laquelle l'ester de tocophérol alkylglycine est un ester d'α-tocophérol alkylglycine.

4. Utilisation selon la revendication 3, **caractérisée en ce que** l'ester d'α-tocophérol alkylglycine est un ester de dl-α-tocophérol alkylglycine.

5. Utilisation selon la revendication 3, **caractérisée en ce que** l'ester d'α-tocophérol alkylglycine est un ester de d-α-tocophérol alkylglycine.

6. Utilisation selon la revendication 1, **caractérisée en ce que** l'ester de tocophérol alkylglycine est un ester de γ-tocophérol alkylglycine.

7. Utilisation selon la revendication 6, **caractérisée en ce que** l'ester de γ-tocophérol alkylglycine est un ester de d-γ-tocophérol alkylglycine.

8. Utilisation selon la revendication 1, **caractérisée en ce que** l'ester de tocophérol alkylglycine est un ester de δ-tocophérol alkylglycine.

9. Utilisation selon la revendication 8, **caractérisée en ce que** l'ester de δ-tocophérol alkylglycine est un ester de d-δ-tocophérol alkylglycine.

10. Utilisation selon la revendication 1, **caractérisée en ce que** l'ester de tocophérol alkylglycine est un ester de tocophérol diméthylglycine.

11. Utilisation selon la revendication 1, **caractérisée en ce que** le sel d'un ester de tocophérol alkylglycine est un sel d'un acide organique ou d'un sel inorganique.

12. Utilisation selon la revendication 11, dans laquelle le sel d'un ester de tocophérol alkylglycine est un hydrochlorure.

13. Utilisation selon la revendication 1, dans laquelle une quantité ajoutée de l'ester de tocophérol alkylglycine et/ou du sel de celui-ci est de 0,1 à 10 % en masse.
